# EUROPEAN PATENT APPLICATION

(11) **EP 2 316 284 A1**
(43) Date of publication of application: **04.05.2011**
(21) Application number: 09173511.8
(22) Date of filing: 20.10.2009
(51) Int. Cl.: A23P 1/04, B01J 13/02, B01J 13/04, B01J 13/10, A61K 9/50

(54) **Barrier composition**

(71) Applicant: Nederlandse Organisatie voor Toegepast -Natuurwetenschappelijk Onderzoek TNO, 2628 VK Delft (NL)
(72) Inventor: Jetten, Jan Matthijs, 3701 JL Zeist (NL); Eversdijk, Jacobus, 5211 XW Den Bosch (NL); van Bommel, Kjeld Jacobus Cornelis, 5625 AG Endhoven (NL); Timmermans, Joahannes Wilhelmus, 6714 GG Ede (NL); ten Cate, Aafke Tessa, 5231 PR's-Hertogenbosch (NL); Slaghek, Theodoor Maximiliaan, 3042 HT Rotterdam (NL)
(74) Representative: Hatzmann, Martin

(57) **Abstract**

The invention is directed to a barrier composition, to a vehicle comprising said barrier composition, to a layer comprising said barrier composition, to a foodstuff comprising said vehicle or layer, to a pharmaceutical or nutraceutical composition comprising said vehicle or layer, to a method for protecting one or more active ingredients, and to the use of said barrier composition.

The barrier composition of the invention comprises:
- a hydrophobic organic phase; and
- biodegradable solid plate-like particles.

## Description

The invention is directed to a barrier composition, to a vehicle comprising said barrier composition, to a layer comprising said barrier composition, to a foodstuff comprising said vehicle or layer, to a pharmaceutical or nutraceutical composition comprising said vehicle or layer, to a method for protecting one or more active ingredients, and to the use of said barrier composition.

In various applications, many active ingredients exhibit significant decrease (of activity) or degradation in their desired properties due to oxidation, hydrolysis and/or other chemical interactions. For example, the food, pharmaceutical, nutraceutical, cosmetics, consumer products, personal care, agrochemical, and chemical industries utilise a significant number of substances that are prone to oxidation. As the substances oxidise, the desired properties of the substances (such as their desired activities) can be decreased or degraded, or even eliminated, or the properties of the substance can be changed entirely to provide undesired properties. In these instances, oxidation protection is required in order to ensure that the substances provide their desired effects at the time of their ultimate use. Similarly, hydrolysis of active ingredients due to the presence of water may lead to degradation or loss of desired properties.

More often than not, oxygen and water from the environment can relatively easily diffuse to and access the active ingredients and cause possibly harmful oxidation and/or hydrolysis. In foodstuffs, nutraceutical compositions and pharmaceutical compositions, such chemical conversions can lead to very undesirable taste deflections. In addition, the oxidation and/or hydrolysis may cause a decrease in activity, or in the extreme a loss of activity. As a consequence, many of the products that contain such oxygen and/or water sensitive active ingredients are usually stored and transported in special packaging wherein the product remains in an oxygen-poor atmosphere surrounded by a film that is less permeable, or even impermeable, to water and/or oxygen.

Protection of active ingredients can also be achieved by incorporating antioxidants in the products. Such antioxidants function as scavengers for oxygen.

Alternatively, it is possible to protect the active ingredients by encapsulating them in a barrier composition. Such barrier compositions are for instance known from WO-A-2006/096051. A disadvantage of the barrier compositions described therein is the fact that the barrier composition comprises water insoluble inorganic particles, such as clay particles. Such water insoluble inorganic particles may give rise to health problems, due to possible accumulation inside the body and possible undesirable body reactions. Moreover, in many cases the inorganic particles will be present in the form of nanoparticles. Many of the effects of inorganic nanoparticles upon administration are yet unknown. It would be desirable to provide a barrier composition that does not require the presence of insoluble inorganic particles.

WO-A-2004/022220 describes a barrier composition microcapsule wherein a polymer material is combined with a structuring agent to provide a higher shelf-life for oxygen and/or water sensitive ingredients. The structuring agent is a material that provides added structure to the microcapsule while decreasing oxygen and/or water permeation through the microcapsule material. Apart from various inorganic structuring materials, this document also mentions the organic structuring materials starch and phospholipids. Although such materials show an oxygen barrier function, this oxygen barrier function is relatively low. Moreover, the inventors realised that this oxygen barrier function decreases considerably at higher water activities. This for instance follows from Forssell et al., Carbohydr. Polym. 2002, 47(2), 125-129 according to which under ambient humidity both amylose and amylopectin films achieved a similar excellent oxygen barrier as a reference commercial ethylene vinyl alcohol film. Forssel *et al*. found that the film permeabilities were determined by water content: below 15 % water both starch films were good oxygen barriers, above 20 % water the barrier property was lost. WO-A-2004/022220 does not disclose a biodegradable plate-like structuring material.

Object of the invention is to provide a barrier composition that advantageously shields oxygen and/or water, in particular oxygen and water.

Further object of the invention is to provide a barrier composition that shields oxygen at a broad range of water activities.

The inventors surprisingly found that these objects can, at least in part, be met by a barrier composition which combines the favourable properties of a water barrier material and an oxygen barrier material.

Accordingly, in a first aspect the invention is directed to a barrier material, comprising:
- a hydrophobic organic phase; and
- biodegradable solid plate-like particles.

The barrier composition advantageously provides protection against possibly detrimental substances from the surroundings, such as oxygen and/or water. The barrier composition can also provide protection against other gases, such as carbon dioxide or ethylene. The barrier composition of the invention comprises a hydrophobic organic phase that mainly provides barrier properties against water. The barrier composition further comprises biodegradable solid particles that mainly provide barrier properties against oxygen. It was found that the barrier composition of the invention in which these two components have been combined exhibits particularly good barrier properties against oxygen at various water activity levels.

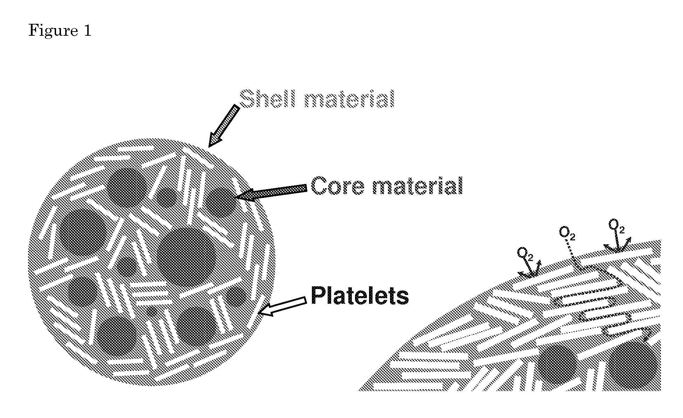

Without wishing to be bound by any particular theory, it is believed that the protection against oxygen and/or water is achieved by a decrease in permeation of these compounds through the hybrid material. This decrease in permeation is believed to be caused by an increase in the length of the pathway for the gas molecules due to the presence of the solid particles. The increase in the length of the pathway for the gas molecules is schematically shown in Figure 1. In addition, the amount of permeable pathways through the barrier composition is decreased, and accordingly total permeation of *e.g.* oxygen through the barrier composition is lowered.

In accordance with the invention, the water barrier resulting from the hydrophobic organic phase ensures the stability of the biodegradable solid particles (*i.e*. protect the particles from dissolution) and prevent swelling of the hybrid composition, which would again decrease the oxygen pathway by increasing the distance between individual particles. Hence, the hydrophobic organic phase and the biodegradable solid particles advantageously interact with each other so as to achieve a synergistic effect, *i.e*. the two components combined create a superior barrier than could be obtained by the components separately.

The term "hydrophobic" as used in this application is meant to refer to being relatively poorly soluble in water or showing minimal swelling in water. The hydrophobic organic phase can suitably comprise one or more selected from the group consisting of proteins (such as caseinate, gluten (or other analogues of wheat-derived polymers), zein (or other analogues of maize-derived polymers), soy protein, and kaferin and hordein and secalin or other plant storage proteins), fatty acids, monoglycerides, diglycerides (and derivatives such as acetylated diglycerides), triglycerides, phospholipids, waxes (such as shellac, bees wax, carnauba wax, candellila wax, and paraffin), natural resins (such as suberin, rosin and derivatives thereof), polysaccharides (such as chitosan, starch, pullulan, gums (including gum Arabic, karaya gum, and xanthan gum), pectinates, xylan, and hemicellulose and derivatives (such as methylcellulose, hydroxypropylcellulose, hydroxymethylpropylcellulose)), and biodegradable polymers (such as polymers and copolymers of lactides, glycolide and hydroxyalkanoates).

In a preferred embodiment, the hydrophobic phase comprises one or more biodegradable biopolymers (and/or derivatives) and/or one or more hydrophobic components having at least one carboxyl group and/or ester group. More preferably, the hydrophobic phase comprises one or more hydrophobic plant storage proteins (called prolamin) such as zein, kaferin, hordein, glutenin or secalin, and one or more hydrophobic components with at least one fatty acid group such as fatty acids (and salts), waxes, and (modified) diglycerides. Even more preferably, the hydrophobic phase comprises one or more zeins.

Apart from hydrophobic components, the hydrophobic organic phase may also comprise one or more less hydrophobic (or even hydrophilic) components, without compromising the overall hydrophobic nature of said phase. Examples include hydrophilic proteins such as chicken protein, whey-protein, or hydrophilic polysaccharides, such as alginate, carrageenan, dextrins.

In an embodiment, the biodegradable solid plate-like particles comprise one or more selected from organic salts, inorganic salts, biopolymer crystals, crystalline saccharides, and layered double hydroxides. The biodegradable solid plate-like particles can comprise one or more organic salts. Examples of organic salts include calcium citrate, and calcium lactate. Examples of inorganic salts include calcium carbonate, magnesium phosphate, and hydroxyapatite. Preferably, the biodegradable solid plate-like particles comprise a crystalline and/or semi-crystalline phase. Such a crystalline and/or semi-crystalline phase is able to contribute to the oxygen barrier function of the particles. In this context, cellulose particles are particularly preferred.

The biodegradable solid particles can be in the micrometer or nanometer scale. The average particle size as measured by transmission electron microscopy is suitably less than 5 µm. Typically, the average particle size of the biodegradable solid particles is not less than 50 nm. Preferably, the average particle size of the biodegradable solid particles is 100-1000 nm, more preferably 100-500 nm.

The biodegradable solid particles have a plate-like form. The term "plate-like particles" as used in this application is meant to refer to mean anisotropic particles whose longest edge dimension (*e.g*. length, diameter or longest axis) greatly exceeds their thickness. For such particles, the aspect ratio between the longest edge dimension (*e.g*. edge-length, diagonal, diameter, *etc*.) and the particle thickness is in the range of 1.5:1 to 5000:1, preferably 5:1 to 2000:1 and most preferably from 10:1 to 1000:1. The inventors found that plate-like particles contribute considerably in forming an effective barrier against oxygen.

The weight ratio between the hydrophobic phase and the biodegradable solid particles in the hybrid matrix barrier composition of the invention can suitably be in the range of 1:99 to 99:1. Preferably, the weight ratio between the hydrophobic phase and the biodegradable solid particles in the hybrid matrix barrier composition is in the range of 1:1 to 1:5.

Preferably, the barrier composition comprises 0.1-75 vol.% of the biodegradable solid plate-like particles basis on total barrier composition volume, more preferably 0.5-70 vol.%, such as 1-65 vol.%, 5-60 vol.% or 10-60 vol.%. It should be noted in this respect that a precise placement of the plate-like particles may allow to use a relatively low amount of the particles in the barrier composition of the invention.

The barrier composition can comprise various additives. Such additives may or may not contribute to the barrier function of the barrier composition. For example, the barrier composition may further include one or more selected from the group of plasticisers, antioxidants, amino acid residues, phospholipids, sugars, cross-linking agents, colorants, and the like. The presence of antioxidants, amino acid residues, phospholipids, and/or sugars in the hybrid matrix barrier composition can aid in enhancing the oxygen and/or water barrier properties.

In a further aspect the invention is directed to a vehicle comprising one or more active ingredients and a barrier composition according to the invention.

Inside the vehicle the one or more active ingredients are well protected from oxygen and/or water by the barrier composition. Examples of active ingredients that can suitably be used in accordance with the invention include unsaturated fatty acids (such as omega-3 fatty acids including α-linolenic acid, eicosapentaenoic acid, docosapentaenoic acid, and docosahexaenoic acid; and omega-6 fatty acids including linoleic acid and arachidonic acid), vitamins, antioxidants, probiotics, peptides, retinol and retinol derivatives (such as β-carotene), retinol palmitate or acetate, flavonoids, lutein, lycopene, zeazanthin, iron salts, copper salts, selenium salts, coenzyme Q10, catalysts (such as Ziegler-Natta and Fischer-Tropsch catalysts), *etc.*

In a preferred embodiment, the vehicle is in the form of a core-shell capsule, in the form of a multi-core particle (particle having multiple cores within a shell matrix, in the form of a multi-shell particle (particle having multiple shells around a single core), or combinations thereof. The shell comprises the barrier composition of the invention and the core comprises the one or more active ingredients. This embodiment is particularly preferable in forming an effective barrier against oxygen and/or water. The core-shell capsules can suitably have an average particle size as measured by transmission electron microscopy of 1-200 µm, preferably 5-25 µm. The thickness of the shell can vary, and can for example be in the range of 0.1-50 µm, preferably in the range of 0.5-10, most preferably 1-5 µm. Core-shell capsules can be prepared by conventional techniques, such as spraying or printing techniques, extrusion, coacervation, fluidised bed coating, polymerisation, emulsions, spinning disk, and jet-cutter.

In another embodiment, the barrier material is in the form of a layer. This can be an individual layer, part of a multilayer, a film, a coating or the like. In particular, the invention includes a film comprising the barrier composition of the invention. Such a film can be advantageously used to cover a product or article which comprises one or more active ingredients that are sensitive to water and/or oxygen. For instance, such films may be useful in packaging.

In a further aspect the invention is directed to a foodstuff comprising the vehicle or the layer of the invention. The invention is also directed to a pharmaceutical or nutraceutical composition comprising the vehicle or layer of the invention.

Since the vehicle of the invention and the layer of the invention provide adequate protection of one or more active ingredients against oxygen and/or water, the product stability and shelf-life of such foodstuffs, or pharmaceutical or nutraceutical compositions is improved as compared to the case wherein the one or more active ingredients are not encapsulated or covered. In addition, the invention allows for improved process stability when preparing the foodstuff, or pharmaceutical or nutraceutical composition. Advantageously, the present invention does not require the presence of insoluble inorganic nanoparticles, which may be harmful for the health of individuals.

In yet a further aspect the invention is directed to a method for protecting one or more active ingredients from oxygen and/or water. The method of the invention comprises encapsulating the one or more active ingredients in a barrier composition according to the invention.

Encapsulating of the one or more active ingredients can be performed by any suitable technique. One method for forming the encapsulated materials of the invention is an atomisation method. Atomisation methods are generally known for forming microencapsulated materials. In the atomisation method, an oil phase and a liquid phase are typically formed and then mixed under high shear to form the desired particle size microcapsules. These microcapsules can be formed around a desired core material, to provide the encapsulated core particles. Such methods can readily be applied to the invention. The atomisation method for preparing core-shell particles is further explained in WO-A-2004/022220. Furthermore, other means of encapsulating include spray chilling, coacervation or complex coacervation, co-extrusion and printing technologies.

In a preferred embodiment, encapsulating of the one or more active ingredients comprises spray-drying, spray cooling, spray-chilling, (spray) freeze drying, fluidized bed-coating system, spinning disk, simple extrusion, double-capillary extrusion, centrifugal extrusion or printing.

In a further aspect the invention is directed to the use of the barrier composition of the invention as a barrier against oxygen and/or water. In addition, the barrier composition of the invention can provide protection against shear and/or temperature at various water activities. Furthermore, the barrier composition of the invention may be applied in order to mask tastes (such as masking bitter tastes or masking deflecting tastes of healthy components).

## Claims

1. Barrier composition, comprising:
- a hydrophobic organic phase; and
- biodegradable solid plate-like particles.

2. Barrier composition according to claim 1, wherein said hydrophobic organic phase comprises one or more selected from the group consisting of proteins, fatty acids, monoglycerides, diglycerides, triglycerides, phospholipids, waxes, natural resins, polysaccharides, and biodegradable polymers.

3. Barrier composition according to claim 1 or 2, wherein said hydrophobic phase comprises
- one of more biodegradable polymers; and/or
- one or more hydrophobic components having at least one carboxyl group and/or ester group.

4. Barrier composition according to any one of claims 1-3, wherein said hydrophobic phase comprises
- one or more hydrophobic plant storage proteins; and
- one or more hydrophobic components with at least one fatty acid group.

5. Barrier composition according to any one of claims 1-4, wherein said hydrophobic phase comprises one or more zeins.

6. Barrier composition according to claim 1-5, wherein said biodegradable solid plate-like particles comprise one or more selected from the group consisting of organic salts, inorganic salts, biopolymer crystals, crystalline saccharides, and layered double hydroxides, preferably one or more selected from the group consisting of calcium citrate, calcium lactate, calcium carbonate, magnesium phosphate, hydrotalcite, hydroxyapatite, amylose and cellulose.

7. Barrier composition according to any one of claims 1-6, wherein said biodegradable solid plate-like particles have an average particle size as measured by transmission electron microscopy of 50-5000 nm, preferably 100-1000 nm, more preferably 100-500 nm.

8. Barrier composition according to any one of claims 1-7, wherein said biodegradable solid plate-like particles have a plate-like form defined by an aspect ratio between the longest edge dimension of the crystal and the crystal thickness of 1.5:1 to 5000:1, preferably 5:1 to 2000:1 and most preferably from 10:1 to 1000:1.

9. Barrier composition according to any one of claims 1-8, wherein said biodegradable solid plate-like particles comprise a crystalline and/or semicrystalline phase.

10. Barrier composition according to any one of claims 1-9, wherein said composition further comprises one or more additives selected from plasticisers, antioxidants, amino acid residues, phospholipids, sugars, cross-linking agents, and colorants.

11. Layer comprising a barrier composition according to any one of claims 1-10.

12. Vehicle comprising one or more active ingredients and a barrier composition according to any one of claims 1-10, said one or more active ingredients preferably selected from the group consisting of anti-oxidants, probiotics, vitamins, peptides, and unsaturated fatty acids, amino acids, metal ions.

13. Vehicle according to claim 12 in the form of a core-shell capsule, wherein said shell comprises said barrier composition and said core comprises said one or more active ingredients.

14. Foodstuff comprising a layer according to claim 11 or a vehicle according to claim 12 or 13.

15. Pharmaceutical or nutraceutical composition, comprising a layer according to claim 11 or a vehicle according to any one of claims 12 or 13.

16. Method for protecting one or more active ingredients from oxygen and/or water, comprising encapsulating said one or more active ingredients in a barrier composition according to any one of claims 1-10.

17. Use of a barrier composition according to any one of claims 1-10 as a barrier against oxygen and/or water.
